# EUROPÄISCHE PATENTSCHRIFT

(11) **EP 1 429 795 B1**
(45) Veröffentlichungstag und Bekanntmachung des Hinweises auf die Patenterteilung: **13.06.2007**
(21) Anmeldenummer: 02777223.5
(22) Anmeldetag: 26.09.2002
(51) Int. Cl.: A61K 36/185

(54) **VERFAHREN ZUR HERSTELLUNG VON EXTRAKTEN AUS PELARGONIUM SIDOIDES UND/ODER PELARGONIUM RENIFORME**
METHOD FOR PRODUCING EXTRACTS OF PELARGONIUM SIDOIDES AND/OR PELARGONIUM RENIFORME
PROCEDE D'OBTENTION D'EXTRAITS DE PELARGONIUM SIDOIDES ET/OU PELARGONIUM RENIFORME

(30) Priorität: 27.09.2001 DE 10147644
(43) Veröffentlichungstag der Anmeldung: 23.06.2004
(73) Patentinhaber: Dr. Willmar Schwabe GmbH & Co. KG, 76227 Karlsruhe (DE)
(72) Erfinder: ERDELMEIER, Clemens, 76139 Karlsruhe (DE); HAUER, Hermann, 76228 Karlsruhe (DE); KOCH, Egon, 76229 Karlsruhe (DE)
(74) Vertreter: Adam, Holger
(86) Internationale Anmeldenummer: PCT/EP2002/010813
(87) Internationale Veröffentlichungsnummer: WO 2003/028746

(56) Entgegenhaltungen:
- KOLODZIEJ H. ET AL: "PELARGONIUM SIDOIDES DC." ZEITSCHRIFT FUR PHYTOTHERAPIE , Bd. 19, Nr. 3, 1998, Seiten 141-151, XP008011448
- HAIDVOGL M. ET AL: " AKUTE BRONCHITIS IM KINDESALTER." ZEITSCHRIFT FUR PHYTOTHERAPIE, Bd. 17, Nr. 5, Oktober 1996 (1996-10), Seiten 300-313, XP008011447

## Beschreibung

Die vorliegende Erfindung betrifft Verfahren zur Herstellung von Extrakten aus *Pelargonium sidoides* und/oder *Pelargonium reniforme.*

*Pelargonium sidoides* ist eine Pflanze, die im südlichen Afrika schon lange als Arzneimittel traditionell verwendet wird bei gastrointestinalen Beschwerden und bei Atemwegserkrankungen einschließlich der Tuberkulose. Der Engländer Stevens entdeckte die Arzneidroge im Jahre 1897 und brachte sie nach England. Dort ergaben sich tatsächlich Behandlungserfolge die dazu führten, dass die Droge von Sechehaye in der Schweiz in die Therapie eingeführt wurde. Sie wurde vor allem in Form von homöopathischen Zubereitungen für mehrwöchige Kuren zur Tuberkulose-Prävention bei prädisponierten Kindern und Erwachsenen verwendet (Kolodziej, H., Kayser, O., Z. Phytother. 19, 141 - 151, (1998)).

Weiterhin sind als pharmakologische Eigenschaften von *Pelargonium sidoides* allgemein antibakterielle Wirkungen gegen einige grampositive (Staphylococcus aureus, Streptococcus pneumoniae, β-hämolysierende Streptokokken) und gramnegative Keime (E. coli, Klebsiella pneumoniae, Proteus mirabilis, Pseudomonas aeroginosa, Hämophilus influenzae) zu erwähnen (Kayser, O., Kolodziej, H., Planta Med. 63, 508 - 510 (1997)).

Die Art *Pelargonium sidoides* wurde lange Zeit hinsichtlich ihrer botanischen Systematik als natürliche Varietät von *Pelargonium reniforme* eingestuft. Deshalb ist in älterer Literatur keine klare Trennung von *Pelargonium sidoides* und *Pelargonium reniforme* vollzogen worden (Kolodziej, H., Kayser, O., Z. Phytother. 19, 141 - 151, (1998)). Aufgrund der großen botanischen Ähnlichkeit von *Pelargonium sidoides* mit der verwandten Spezies *Pelargonium reniforme* ist davon auszugehen, daß die in der Regel aus Wildsammlungen stammenden Wurzeln von *Pelargonium sidoides* wechselnde Anteile an *Pelargonium reniforme* enthalten.

Hauptinhaltsstoffe von *Pelargonium sidoides* Wurzeln sind Proanthocyanidine, die vorwiegend aus Catechin- und Gallocatechineinheiten aufgebaut sind (Kayser, O., Dissertation, Fachb. Pharm. Freie Univ. (FU) Berlin, S. 23 - 24 und 51 - 55 (1997)). Weiterhin kommen einfache Cumarine vor, mit in der Regel hohem Oxygenierungsgrad an der Cumaringrundstruktur. Dies scheint ein für *Pelargonium* relativ typisches Strukturmerkmal zu sein (Kayser, O., Kolodziej, H., Phytochemistry 39, 1181 - 1185 (1995); Latte, K. P., Kayser, O., Tan, N., Kaloga, M., Kolodziej, H., Z. Naturforsch. 55c, 528 - 533 (2000)).

Extrakte aus Wurzeln von *Pelargonium sidoides* und/oder *reni*forme wurden bisher üblicherweise z.B. durch einfache Mazeration unter Verwendung von Aceton / Wasser 4/1 (Kayser, 0., Kolodziej, H., Phytochemistry 39, 1181 - 1185 (1995)) bzw. reinem Methanol im Soxhletverfahren (Bladt, S.; Wagner, H. Dtsch. Apoth.-Ztg. 128, 292 - 296 (1988)) hergestellt. Zur therapeutischen Anwendung wurden auch rein wässrige Extrakte verwendet (Kolodziej, H., Kayser, O., Z. Phytother. 19, 141 - 151 (1998)). Im Handel erhältlich ist ein mit Glycerin verdünnter ethanolischer Auszug aus *Pelargonium reniforme*/*sidoides* (Rote Liste 2001).

Die Nachteile bisheriger Extraktionsverfahren liegen in relativ niedrigen Ausbeuten, bzw. starker Temperaturbelastung (Soxhlet).

Der vorliegenden Erfindung liegt die Aufgabe zugrunde, ein verbessertes schonendes Verfahren zur Herstellung von Extrakten aus *Pelargonium sidoides* und *Pelargonium reniforme* bereitzustellen, mit dem Extrakte in höheren Ausbeuten erhalten werden, wobei gleichzeitig jedoch die Wirkung der Extrakte verbessert werden soll.

In der vorliegenden Erfindung wird zur Lösung dieser Aufgaben ein Verfahren zur Herstellung eines Extraktes aus *Pelargonium sidoides* und/oder *Pelargonium reniforme* beschrieben, dadurch gekennzeichnet, dass die Wurzeln von *Pelargonium sidoides* und/oder *reniforme* entweder einer Perkolation oder einer zweistufigen Mazeration unterworfen werden. Die Perkolation bzw. die zweifache Mazeration werden vorzugsweise mit wässrig ethanolischen Lösungsmitteln durchgeführt. Ein bevorzugtes Lösungsmittel ist 10-92 Gew.-% Ethanol und insbesondere 10-60 Gew.-% Ethanol. Die Perkolation kann auch dadurch erfolgen, dass die Wurzeln mit dem Lösungsmittel angemaischt werden, wobei das Anmaischen und das anschließende eigentliche Perkolieren mit gleich oder unterschiedlich konzentriertem wässrigen Ethanol erfolgen kann. Im letzteren Fall erfolgt das Anmaischen vorzugsweise mit einem relativ höher konzentrierten wässrigen Ethanol, wie 35 Gew.-% Ethanol und die anschließende Perkolation mit einem relativ weniger konzentrierten wässrigen Ethanol, wie 5 Gew.-% Ethanol z.B. im Verhältnis 2:8, damit die durchschnittliche Konzentration an verwendetem Ethanol in den angegebenen Bereichen (10-92 Gew.-%, vorzugsweise 10-60 Gew.-%) liegt. Im vorstehenden Beispiel ergibt sich eine gewichtete durchschnittliche Konzentration von 11 Gew.-% Ethanol ([35 x 2 + 5 x 8]/ 10 Gew.-%). Die erfindungsgemäß erhaltenen Extrakte weisen nach dem Trocknen einen Gesamtphenolgehalt von mindestens 15%, vorzugsweise mindestens 18% auf. Derartige Extrakte sind besonders gut zur Behandlung von akuten und chronischen Entzündungserkrankungen und Infektionen geeignet.

Die Trockenextraktausbeuten bei der erfindungsgemäßen Perkolation (Beispiele 1 bis 4) und bei der erfindungsgemäßen zweistufiger Mazeration mit 11 bis 60 Gew.-% Ethanol (Beispiele 5 bis 8) sind etwa doppelt so hoch wie bei einfacher Mazeration entsprechend der Vorschrift des DAB (Vergleichsbeispiel 1).

Bei Extraktionsversuchen getrockneter Wurzeln von *Pelargonium sidoides* mit Wasser-Ethanol-Gemischen wurde überraschenderweise festgestellt, dass mit steigendem Ethanolgehalt im Extraktionsmittel der Gehalt an Gesamtphenolen im resultierenden Extrakt stetig ansteigt und nicht wie zu erwarten gewesen wäre bei mittleren Ethanolkonzentrationen ein Maximum durchläuft. Sowohl bei der Perkolation (Beispiele 1 bis 4) als auch bei der zweistufigen Mazeration (Beispiele 5 bis 9) sind die Gehalte an Gesamtphenolen gegenüber der einfachen Mazeration (Vergleichsbeispiel 1) erhöht, wobei je nach Extraktionslösungsmittel der Faktor 3 erreicht werden kann.

Parallel zu den Gesamtphenolgehalten steigt auch das antioxidative Potential der Extrakte an. Dabei sind die erfindungsgemäßen Extrakte den Vergleichsextrakten nach Vergleichsbeispielen 1 und 2 überlegen. Da die Gesamtcumaringehalte in den Extrakten unabhängig vom Verfahren und vom Lösungsmittel praktisch konstant bleiben, ist davon auszugehen, dass die Cumarine nur einen unwesentlichen Beitrag zur antioxidativen Wirkung leisten.

Aus dem Vergleich der Beispiele 1 (Anmaischen mit 35 Gew.-% Ethanol und Perkolieren mit 5 Gew.-% Ethanol), 2 (Anmaischen mit 11 Gew.-% Ethanol und Perkolieren mit 11 Gew.-% Ethanol) und 4 (Perkolieren mit 11 Gew.-% Ethanol ohne vorhergehendes Anmaischen) ergibt sich für den Extrakt gemäß Beispiel 1 die höchste Ausbeute und für den Extrakt gemäß Beispiel 4 den höchsten Gesamtphenolgehalt. Das antioxidative Potential liegt für die Extrakte gemäß der Beispiele 1 und 4 etwas über dem des Extraktes gemäß Beispiel 2. Auch stimmen die IC₅₀-Werte für die Elastase-Hemmung bei den Extrakten gemäß Beispiel 1 und 4 nahezu überein, wohingegen der IC₅₀-Wert für den Extrakt gemäß Beispiel 2 vergleichsweise hoch bei 8,4 µg /ml liegt. Demnach sind die Verfahren gemäß der Beispiele 1 und 4 gegenüber dem Verfahren gemäß Beispiel 2 bevorzugt. D.h. die Perkolation ohne Anmaischen und die Perkolation mit Anmaischen durch höher konzentriertes Ethanol und Perkolieren mit weniger konzentriertem Ethanol sind gegenüber einem Verfahren, bei dem das Anmaischen und die Perkolation mit gleich konzentriertem Ethanol erfolgt, bevorzugt.

Das im Handel befindliche Präparat aus *Pelargonium reniforme*/*sidoides* wird vor allem zur Behandlung von akuten und chronischen Infektionen, speziell der Atemwege und des Hals-Nasen-Ohrenbereichs, eingesetzt. Als wesentliche pharmakologische Effekte des Produkts werden antimikrobielle Wirkungen und/oder die Stimulation der unspezifischen Immunabwehr angesehen. An vorderster Front der Immunabwehr gegenüber Krankheitserregern und insbesondere Bakterien stehen neutrophile Granulozyten. Die Stimulation dieser Leukozyten, z.B. im Verlauf der Phagozytose von Mikroorganismen, geht mit einem erhöhten Sauerstoffverbrauch und der Bildung von hochreaktiven Sauerstoffverbindungen (z.B. Superoxid, Wasserstoffperoxid) einher, mit deren Hilfe diese Zellen Infektionserreger abtöten. Bei einer überschießenden Abwehrreaktion und im Verlauf von chronischen Infektionen können die von phagozytierenden Zellen freigesetzten reaktiven Sauerstoffspezies aber auch Gewebeschäden hervorrufen. Substanzen mit antioxidativen Eigenschaften können diese unerwünschten Nebenwirkungen verhindern und dadurch den Heilungsprozess verbessern und beschleunigen (Cuzzocrea et al., Pharmacol. Rev. 53, 135 (2001)). Da die antioxidativen Eigenschaften vom Gesamtphenolgehalt abhängig sind, ergibt sich für die erfindungsgemäßen Extrakte damit eine eindeutige Überlegenheit gegenüber den Vergleichsextrakten.

Neben reaktiven Sauerstoffspezies nutzen phagozytierende Zellen auch proteolytische Enzyme, um Bakterien abzutöten. Zu den wichtigsten und potentesten von Leukozyten gebildeten und freigesetzten Proteasen zählt die Elastase. Dieses Enzym ist aufgrund seines destruktiven Einflusses auf das Wirtsgewebe von pathophysiologischer Bedeutung und Elastase-Inhibitoren haben deshalb eine breite therapeutische Anwendungen bei akuten und chronischen Entzündungskrankheiten, wie z.B. bei chronischer Bronchitis und anderen Atemwegserkrankungen, Lungenemphysem, cystischer Fibrose, rheumatoider Arthritis, Myocarditis etc. (Fujie et al., Eur. J. Pharmacol., 374, 117 (1999)).

Es wurde jetzt überraschend beobachtet, dass Extrakte aus Pe*largonium sidoides* und/oder *reniforme* über potente Hemmaktivitäten gegenüber der menschlichen Leukozytenelastase (HLE) verfügen und dass diese Wirkung ebenfalls mit dem Gehalt der Extrakte an Gesamtphenolen korreliert. Der duale Wirkmechanismus aus antioxidativen Eigenschaften und Hemmung der HLE gilt als besonders vorteilhaft, da er die zwei wichtigsten pathogenetischen Faktoren, die zur Schädigung des körpereigenen Gewebes bei chronischen Entzündungsprozessen beitragen, ursächlich beeinflusst (Portevin et al., J. Med. Chem. 40, 1906 (1997)).

Die erhaltenen Extrakte können zusammen mit den üblichen pharmazeutischen Hilfsstoffen zu pharmazeutischen festen Zubereitungen wie Kapseln, Tabletten, überzogene Tabletten oder Kautabletten verarbeitet werden. Als pharmazeutische Hilfsstoffe werden übliche Füll-, Binde-, Spreng-, Schmiermittel sowie Geruchs- und Geschmacksstoffe und Überzugsmittel für überzogene Tabletten verwendet. Als pharmazeutische Hilfsstoffe zur Herstellung von Weichgelatinekapseln werden übliche Öle und Fette als Füllmassen für Weichgelatinekapseln verwendet. Die erhaltenen Extrakte können zusammen mit pharmazeutisch üblichen Hilfsstoffen zu pharmazeutischen flüssigen Zubereitungen wie Lösungen, Sprays und Suspensionen verarbeitet werden. Als pharmazeutische Hilfsstoffe werden übliche Lösungsmittel, Lösungsvermittler, Stabilisatoren sowie Geruchs- und Geschmacksstoffe verwendet.

Die Erfindung wird anhand folgender nicht einschränkender Beispiele erläutert. Um vergleichbare analytische und pharmakologische Daten zu erhalten, wurde bei allen Beispielen zum Trockenextrakt getrocknet. Die Trockenextrakte repräsentieren jedoch die zugrunde liegenden Extraktlösungen, welche ebenfalls Gegenstand der Erfindung sind.

### Beispiele 1 bis 4 - Perkolation mit verschiedenen Extraktionslösungsmitteln

### Beispiel 1

1 kg getrocknete Wurzeln von *Pelargonium sidoides* wurden 24 h mit 2 kg 35 Gew.-% EtOH vorgequellt und danach in eine Glassäule überführt. Dann wurde 10 h mit 8 kg 5 Gew.-% EtOH perkoliert, anschließend der Drogenrückstand leicht ausgepresst, der Rohextrakt über Seitz Supra 1500 filtriert und zur Trockne eingeengt.

### Beispiele 2 und 3

40 g getrocknete Wurzeln von *Pelargonium sidoides* wurden 22 h mit 80 g Extraktionslösungsmittel (11 Gew.-% bzw. 35 Gew.-% Ethanol) vorgequellt und danach in eine Glassäule überführt. Dann wurde 3 bis 4 h mit 320 g des obigen Extraktionslösungsmittels perkoliert, anschließend der Drogenrückstand leicht ausgepresst und der klare Rohextrakt zur Trockne eingeengt.

### Beispiel 4

40 g getrocknete Wurzeln von *Pelargonium sidoides* wurden in eine Glassäule gefüllt und 8 h mit 400 g 11 Gew.-% Ethanol perkoliert. Anschließend wurde der Drogenrückstand leicht ausgepresst, der Rohextrakt über Seitz Supra 1500 filtriert und zur Trockne eingeengt.

| Beisp. Nr. | Extraktions-Lösungsm. Gew.-% EtOH | Ausbeute (%) | Gesamtphenole (%) | Gesamtcumarine (%) | Antioxidatives Potential µg Trolox / mg | Elastase-Hemmung IC₅₀ (µg/ml) |
|---|---|---|---|---|---|---|
| 1 | 11(35%/5%) | 18.4 | 17.47 | 3.18 | 279 | 5.7 |
| 2 | 11 | 15.3 | 17.09 | 3.14 | 237 | 8.4 |
| 3 | 35 | 17.2 | 24.16 | 2.84 | 311 | 6.2 |
| 4 | 11 | 17.8 | 21.79 | 2.69 | 273 | 5.9 |

### Vergleichsbeispiel 1 - Mazeration nach DAB

20 g getrocknete Wurzeln von *Pelargonium sidoides* wurden entsprechend der Mazerationsvorschrift für Extrakte nach DAB 10 mit 140 ml 11 Gew.-% Ethanol bei Raumtemperatur über 5 Tage unter gelegentlichem Umschütteln und nachfolgender Lagerung über 5 Tage bei kühler Temperatur extrahiert. Anschließend wurde filtriert und die Extraktlösung getrocknet.

### Beispiele 5 bis 9 und Vergleichsbeispiel 2 - Mazeration mit verschiedenen Extraktionslösungsmitteln

20 g getrocknete Wurzeln von *Pelargonium sidoides* wurden mit 140 g Extraktionslösungsmittel (Ethanol/Wasser in der unten angegebenen Zusammensetzung bzw. Wasser (Vergleichsbeispiel 2)) bei 50 °C während 30 Minuten mazeriert. Anschließend wurde die Extraktlösung abfiltriert und der Drogenrückstand ein zweites Mal in der gleichen Weise mazeriert. Nach der Fest / Flüssigtrennung (Filtration) wurden die Extraktlösungen vereinigt und getrocknet.

| Beisp. Nr. | Extraktions-Lösungsm. Gew.-% EtOH | Ausbeute (%) | Gesamtphenole (%) | Gesamtcumarine (%) | Antioxidatives Potential µg Trolox / mg | Elastase-Hemmung IC₅₀ (µg/ml) |
|---|---|---|---|---|---|---|
| Vergleich 1 | 11 | 8.0 | 12.37 | 3.14 | 211 | >10 |
| Vergleich 2 | 0 (Wasser) | 13.8 | 14.07 | 2.69 | 234 | 9.0 |
| 5 | 11 | 16.2 | 18.72 | 2.65 | 271 | 6.0 |
| 6 | 20 | 16.5 | 20.10 | 2.42 | 304 | 5.0 |
| 7 | 35 | 17.6 | 24.13 | 2.43 | 353 | 4.2 |
| 8 | 60 | 15.7 | 27.53 | 2.63 | 366 | 5.9 |
| 9 | 90 | 7.6 | 36.18 | 2.93 | 508 | 4.5 |

### Analytische und pharmakologische Methoden

### Bestimmung der Gesamtphenole

Die Bestimmung der Gesamtphenole erfolgt in Analogie zur Arzneibuchmethode für Gerbstoffe (DAB 2000) photometrisch nach Umsetzung mit Molybdat-Wolframat-Reagenz. Dazu wird der Extrakt in wässrigem Ethanol gelöst, mit Natriumcarbonat-Lösung alkalisiert und mit Molybdat-Wolframat-Reagenz versetzt. Nach Zentrifugation wird die Extinktion der überstehenden Lösung bei 720 nm gegen Wasser gemessen. Die Berechnung erfolgt auf Epicatechin.

### Bestimmung der Gesamtcumarine

Die Bestimmung der Gesamtcumarine erfolgt mittels HPLC über eine RP-18 Säule. Als mobile Phase wird ein Acetonitril / Wasser / Phosphorsäure-Gradient (10:990:4 → 205:795:4) verwendet. Detektiert wird im UV bei 330 nm. Die Berechnung der einzelnen Cumarin-Peaks erfolgt als Scopoletin.

### Bestimmung des antioxidativen Potentials

Das angewandte Verfahren von Shi, H. und Niki, E. (Lipids 33, 365 (1998)) beruht auf der photometrischen Messung der mit der Reduktion eines stabilen Radikals (Galvinoxyl) einhergehenden Extinktionsänderung.
Dazu werden 2.69 mg Galvinoxyl in 500 ml Ethanol gelöst (12,75 µmol/l). Davon werden 8 ml mit 200 µl Probe bzw. Kalibrierlösung (s. u.) versetzt und 20 min bei Raumtemperatur im Dunkeln inkubiert. Danach wird am Photometer bei 428 nm gemessen.
Kalibrierlösung: 0,954 mg Trolox (6-Hydroxy-2,5,7,8-tetramethylchroman-2-carbon-säure) werden in 25 ml Ethanol / Wasser 1/1 gelöst. Aus dieser Stammlösung werden 1/25, 1/20, 1/10, 1/5 sowie 1/2 Verdünnungen in Ethanol / Wasser 1/1 hergestellt und die Absorption bei 428 nm gemessen.

### Bestimmung der Elastase-Hemmung

Der Einfluss von Extrakten auf die peptidolytische Aktivität von HLE (E.C. 3.4.21.37; Sigma) wurde bei Raumtemperatur in 50 mM Tris-HCl (pH 8) mit 50 mM NaCl und 0,01 % Brij 35 bestimmt. HLE (50 µl, 200 mU/ml) wurde für 15 Minuten mit 50 µl der Extrakte in einer Mikrotiterplatte inkubiert. Die Enzymreaktion wurde anschließend durch Zugabe von 50 µl Substrat (Methoxysuccinyl-Ala-Ala-Pro-Val-p-nitroanilide; 1 mM; Sigma) gestartet und über einen Zeitraum von 10 Minuten in einem Mikroplatten-Photometer bei 405 nm gemessen (Thermomax, Molecular Devices). Die Hemmung der Enzymaktivität wurde im Vergleich zu einer identischen Lösungsmittelkontrolle (DMSO) berechnet.

## Patentansprüche

1. Verfahren zur Herstellung eines Extraktes aus *Pelargonium sidoides* und/oder *Pelargonium reniforme,* **dadurch gekennzeichnet, dass** die Wurzeln aus *Pelargonium sidoides* und/oder *reniforme* entweder
a) einer Perkolation mit einem wässrig-ethanolischen Lösungsmittel unterworfen werden, der Drogenrückstand gegebenenfalls leicht ausgepresst und der rohe Extrakt gegebenenfalls filtriert wird, oder
b) einer zweistufigen Mazeration mit einem wässrig-ethanolischen Lösungsmittel unterworfen werden, wobei nach der ersten Mazeration die Extraktlösung abfiltriert und der Drogenrückstand ein zweites Mal mazeriert wird und die Extraktlösungen nach der Fest/Flüssigtrennung vereinigt werden.

2. Verfahren nach Anspruch 1, wobei der in Schritt a) oder b) erhaltene flüssige Extrakt getrocknet wird, um einen Trockenextrakt zu erhalten.

3. Verfahren nach Anspruch 1 oder 2, wobei bei der Perkolation vor dem eigentlichen Perkolieren ein Anmaischen mit einem wässrig-ethanolischen Lösungsmittel erfolgt.

4. Verfahren nach Anspruch 3, wobei das Anmaischen und das Perkolieren mit unterschiedlich konzentriertem wässrigen Ethanol erfolgt.

5. Verfahren nach einem der Ansprüche 1 bis 3, wobei das wässrig-ethanolische Lösungsmittel 10-92 Gew.-% Ethanol ist.

6. Verfahren nach einem der Ansprüche 1 bis 3, wobei das wässrig-ethanolische Lösungsmittel 10-60 Gew.-% Ethanol ist.

7. Verfahren nach Anspruch 4, wobei die gewichtete durchschnittliche Konzentration des beim Anmaischen und beim Perkolieren verwendeten wässrigen Ethanols im Bereich von 10-92 Gew.-%, vorzugsweise 10-60 Gew.-% liegt.

8. Verfahren nach Anspruch 4, wobei für das Anmaischen 35 Gew.-% Ethanol und das Perkolieren 5 Gew.-% Ethanol im Verhältnis 2:8 verwendet wird.

## Claims

1. Method for producing an extract from *Pelargonium sidoides* and/or *Pelargonium reniforme,* **characterized in that** the roots of *Pelargonium sidoides* and/or *reniforme* are either
a) subjected to percolation using an aqueous ethanolic solvent, the drug residue is optionally slightly squeezed and the raw extract is optionally filtered, or
b) subjected to a two-step maceration using an aqueous ethanolic solvent, wherein the extract solution is filtered after the first maceration and the drug residue is macerated a second time and the extract solutions are combined after a solid/liquid separation.

2. Method according to claim 1, wherein the liquid extract obtained in step a) or b) is dried to obtain a dry extract.

3. Method according to claim 1 or 2, wherein an initial mashing using an aqueous ethanolic solvent is carried out before the actual percolation step in the case of the percolation.

4. Method according to claim 3, wherein the initial mashing and the percolation are carried out using aqueous ethanol having different concentrations.

5. Method according to any one of claims 1 to 3, wherein the aqueous ethanolic solvent is 10-92% by weight ethanol.

6. Method according to any one of claims 1 to 3, wherein the aqueous ethanolic solvent is 10-60% by weight ethanol.

7. Method according to claim 4, wherein the weighted average concentration of the aqueous ethanol used for initially mashing and percolating is within a range of 10-92% by weight, preferably 10-60% by weight.

8. Method according to claim 4, wherein 35% by weight ethanol is used for initially mashing and 5% by weight ethanol is used for percolating in a ratio of 2:8.

## Revendications

1. Procédé de production d'un extrait de *Pelargonium sidoides* et/ou de *Pelargonium reniforme,* **caractérisé en ce que** les racines de *Pelargonium sidoides* et ou *reniforme*
a) sont soumises à une percolation avec un solvant aqueux éthanolique, le résidu médicamenteux est éventuellement légèrement pressé et l'extrait brut est éventuellement filtré, ou
b) sont soumises à une macération en deux étapes avec un solvant aqueux étanolique, la solution d'extraction étant filtrée après la première macération et on fait macérer une deuxième fois le résidu médicamenteux et les solutions d'extraction sont réunies après la séparation en phases solide/liquide.

2. Procédé selon la revendication 1, l'extrait fluide obtenu à l'étape a) ou à l'étape b) étant séché pour obtenir un extrait sec.

3. Procédé selon la revendication 1 ou 2, un foulage avec un solvant aqueux éthanolique avant la percolation effective étant effectué lors de la percolation.

4. Procédé selon la revendication 3, le foulage et la percolation s'effectuant avec de l'éthanol aqueux à différentes concentrations.

5. Procédé selon l'une quelconque des revendications 1 à 3, le solvant aqueux éthanolique étant de l'éthanol de 10 à 92 % en poids.

6. Procédé selon l'une quelconque des revendications 1 à 3, le solvant aqueux éthanolique étant de l'éthanol de 10 à 60 % en poids.

7. Procédé selon la revendication 4, la concentration moyenne pondérée de l'éthanol aqueux utilisé lors du foulage et de la percolation se situant dans une plage allant de 10 à 92 % en poids, de préférence, de 10 à 60 % en poids.

8. Procédé selon la revendication 4, sachant que l'on utilise pour le foulage, de l'éthanol à 35 % en poids et pour la percolation, de l'éthanol à 5 % en poids en un rapport de 2 : 8.
